# EUROPEAN PATENT SPECIFICATION

(11) **EP 2 958 482 B1**
(45) Date of publication and mention of the grant of the patent: **24.06.2020**
(21) Application number: 14753933.2
(22) Date of filing: 19.02.2014
(51) Int. Cl.: A61B 1/05, G02B 27/00, A61B 1/00, A61B 1/002, G02B 23/24, G02B 23/02

(54) **ENDOSCOPE WITH PUPIL EXPANDER**
ENDOSKOP MIT EINER PUPILLENDEHNVORRICHTUNG
ENDOSCOPE À ÉCARTEUR DE PUPILLE

(30) Priority: 19.02.2013 US 201361766576 P
(43) Date of publication of application: 30.12.2015
(73) Proprietor: Steris Instrument Management Services, Inc., Birmingham, AL 35222 (US)
(72) Inventor: ZOBEL, Jurgen, Pembroke Pines, FL 33028 (US)
(74) Representative: Range, Christopher William
(86) International application number: PCT/US2014/017153
(87) International publication number: WO 2014/130547

(56) References cited:
- EP-A2- 0 710 039
- WO-A1-97/27798
- JP-A- 2010 128 354
- KR-A- 20110 047 690
- US-A1- 2002 161 278
- US-A1- 2012 075 448
- US-A1- 2012 075 448
- US-B1- 6 471 642
- US-B1- 8 149 270
- US-B1- 8 149 270

## Description

### Technical Field

The present invention is directed to an optical system for an endoscope. More particularly, the present invention is directed to an optical system for a monoscopic endoscope, the optical system including a pupil expander assembly for creating two stereoscopic images.

### Background of Invention

Technical and medical endoscopes are delicate optical instruments that are introduced in technical and human cavities to inspect the interior of the cavities. Such endoscopes can be rigid endoscopes containing a lens system, flexible endoscopes containing a flexible image guiding bundle or video endoscopes.

Endoscopes have a small diameter of a few millimeters and are often several hundreds of millimeter long. Endoscopes contain an outer tube and an inner tube. The space between the outer tube and the inner tube is filled with illumination fibers that guide externally created light inside the cavities. Inside the inner tube is an optical system that relays an image of the cavity from the distal tip of the endoscope back to the proximal end of the endoscope. This relayed image can be observed at the proximal end by the operator's eye, or a video camera can capture the image.

When the first endoscopes with such lens systems where introduced at the beginning of the 20th century surgeons and designers contemplated stereo endoscopic systems. Stereoscopic systems in general produce two images that show an object space from slightly different perspectives, namely, a left image and a right image. The human brain is capable of merging these two slightly different images and transferring the so-called disparity of the two images, via information about depth, and creates a three-dimensional impression.

Endoscopes with a single optical system, i.e., monoscopic endoscopes, create three-dimensional information about the object space. Objects in different distances appear in different sizes, and overlapping of objects informs the user which object is in the front and which is in the back. In this way, endoscopic instruments inserted through and viewed by an endoscope can be observed by the user moving back and forth in the object space. For many surgical applications in endoscopy this three dimensional information is sufficient, and there is no need for stereoscopic vision. However, with an increasing number of complex surgical procedures being performed and observed through endoscopes, the control of the position of endoscopic instruments has become more crucial for some surgeries. Such complex surgeries are now often performed with robotic assistance, and the position of the instruments is controlled by stereo endoscopic observations.

Stereo endoscopes typically include two optical systems that are difficult to assemble and align and expensive to manufacture. For these reasons, stereo endoscopes having two optical systems are not economically or technically feasible for certain applications. In those instances, it is preferred use a monoscopic endoscope that is modified to provide a stereo endoscopic capability by separating the pupil of the single optical system into a left portion and a right portion. A shortcoming of endoscopes of this type of modified endoscope, however, is that the stereoscopic eye base of such endoscopes is about half the entrance pupil of the endoscope. Such entrance pupils have a diameter of a few tenth of a millimeter. This results in a reasonable stereoscopic working distance of only around one mm or less than one mm. The present invention addresses the small stereoscopic eye base problem that is associated with monoscopic endoscopes that have been modified as explained above by providing an endoscope having a single optical system with a pupil expander.

WO-A-97/27798 relates to a stereoscopic endoscope having two laterally separated objective lens elements.

EP-A-0710039 relates to a small video camera apparatus.

US6,471,642-B relates to a rigid endoscope optical system which can obtain both a wide angle image for finding an organ and a high resolution image used for precise treatment.

US-A-2012/0075448 relates to a stereoscopic image capturing objective optical system and endoscope.

US8,149,270-B relates to a high resolution endoscope employing a single light sensor array and a lenticular lens layer located at a proximal end of the endoscope.

### Summary of the Invention

According to the invention there is provided an optical system according to claim 1.

The present invention, which is defined in the appended claims, is directed to stereo endoscopes that include a single optical system or train but that have an extended distance between the left image side and the right image side of the entrance pupil. According to the present invention, the optical train of a unitary optical system is separated into a left optical train and a right optical train. The separation into left and right optical trains is achieved by splitting the entrance pupil into a left and right pupil half.

To additionally achieve a significant stereoscopic disparity the distance between the left and right pupil half is expanded. The separation of the entrance pupil and the expansion of the left and right pupil halves are achieved by a distally located prism block. This prism block consists of a pair of rhomboidal prisms that are located exactly at the entrance pupil inside the endoscope objective. A front lens group is assembled in front of each of the two rhomboidal prisms. Such stereo endoscopes with expanded pupil halves enable to build stereo endoscopes with very small diameter or stereo endoscopes for special surgical applications.

The information for the left and right perspective of the endoscopic image is related to the left and right half of the entrance pupil. The circular entrance pupil of such a stereo endoscope can be optically separated, and the distance of the two halves of the entrance pupil can be expanded and the stereoscopic effect can be increased.

According to one aspect of the invention, there is provided a prism block that is located at the surface of the entrance pupil. The prism block includes of a pair of rhomboidal prisms that are located exactly at the entrance pupil inside the endoscope objective. The two rhomboidal prisms touch at one side and divide the entrance pupil in half. The rhomboidal prisms are arranged so that the entrance pupil of the endoscope sits exactly at the exit surface of the rhomboidal prisms. In this manner, one rhomboidal prism deflects all rays going through the left portion of the entrance pupil to the left and transfers this half of the entrance pupil to the left. The other rhomboidal prism deflects all rays going through the right portion of the entrance pupil to the right and transfers this half of the entrance pupil to the right. The deflection angles of the two rhomboidal prisms have a slightly different angle so that the optical axes of the left and right rhomboidal prisms converge in the working distance of the stereo endoscope.

The optical system further includes the two front lens groups that are aligned in front of the left and the right halves of the entrance pupil. The two front lens groups are glued on a wedged glass plate so that the optical axes of the negatives correspond to the converging optical axes exiting the two rhomboidal prisms. The two units, each consisting of one of the negative lens groups and one of the wedged glass plates, are then aligned under optical control so that the optical fields of the two optical trains overlap in the working distance.

According to another aspect of the invention, the two rhomboidal prisms are fixed on a triangle prism, and the combination of these three prisms is glued to a glass window. The glass window and the three prisms form a prism block that can be easily aligned in front of the entrance pupil of the stereo endoscope and fixed to a piano surface of the endoscope objective.

According to another aspect of the invention, at a proximal end of the described stereo endoscopes, the left half of the exit pupil contains the information gathered through the expanded left side of the entrance pupil and the right half of the exit pupil contains the information gathered through the expanded right side of the entrance pupil. This information in the exit pupil of the stereo endoscope can be separated by a prism block including two rhomboidal prisms placed in the tip of a stereo endoscope camera.

According to yet another aspect of the invention, the prism block comprising two rhomboidal prisms can be used to separate the entrance pupil of a video endoscope objective. In this embodiment, the prism block is positioned at the aperture which represents the entrance pupil of the video endoscope objective to create two halves of the aperture. On the video chip the two images from the left and right perspective are overlaid. The two images are alternatingly blocked by a LCD shutter thereby allowing the left and right image to be read out from the chip separately. Such a LCD shutter can be integrated in the prism block by substituting the prior described glass window holding the prisms with a pair of LCD shutters. If the image signal for the left and the right image is then processed separately, the stereo video signal can be displayed with the stereo endoscope system described in WO 2014012103, titled "Stereo Endoscope System". In particular, the stereo endoscope of the present invention can be mechanically and optically coupled to the stereo video cameras described in WO 2014012103 A1.

The described prism block can also be used to separate the exit pupil of stereo endoscopes in a left and right half and expand the distance between the optical axes of the two halves to adapt the stereo endoscope to the optical axes of any stereo endoscope camera.

### Brief Description of the Drawings

FIG. 1 is a sectional view of a stereo endoscope optical system including two optical trains in accordance with the prior art.
FIG. 1A is a front view of the stereo endoscope optical system of FIG. 1 depicting a pair of entrance pupils.
FIG. 2 is a sectional view a stereo endoscope optical system including a single optical train in accordance with the prior art.
FIG. 2A is a front view of the stereo endoscope optical system of FIG. 2 depicting a pair of entrance pupils.
FIG. 3 is a sectional view of a stereo endoscope optical system including a single optical train and a pupil expander assembly in accordance with the present invention.
FIG. 3A is a front view of the stereo endoscope optical system of FIG. 3 depicting an entrance pupil of the optical system divided and separated into a left half and a right half.
FIG. 4 is a sectional view of the stereo endoscope optical system of FIG. 3 displaying right perspective and left perspective views of a point in an object field of the optical system.
FIG. 5 depicts a pair of negative front lens, a pair of rhomboidal prisms, the divided entrance pupil and a plano-convex lens of the stereo endoscope optical system of FIG. 3.
FIG. 6 depicts convergent optical axes of the pair of rhomboidal prisms of the stereo endoscope optical system of FIG. 5.
FIG. 7 depicts the pair of rhomboidal prisms of the stereo endoscope optical system of FIG. 5 assembled into a prism block assembly.
FIG. 8 is magnified view of the stereo endoscope optical system of FIG. 3.
FIG. 9 is a sectional view of a stereo endoscope optical system, where the pair of rhomboidal prisms of the stereo endoscope sits in front of a deflecting prism.

### Detailed Description of Preferred Embodiment and Figures

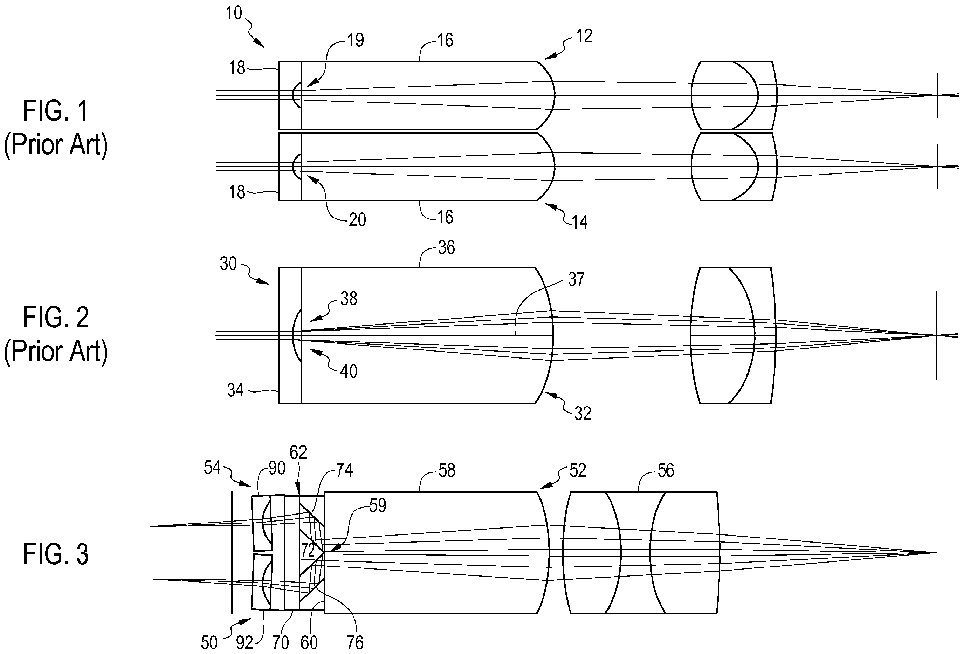

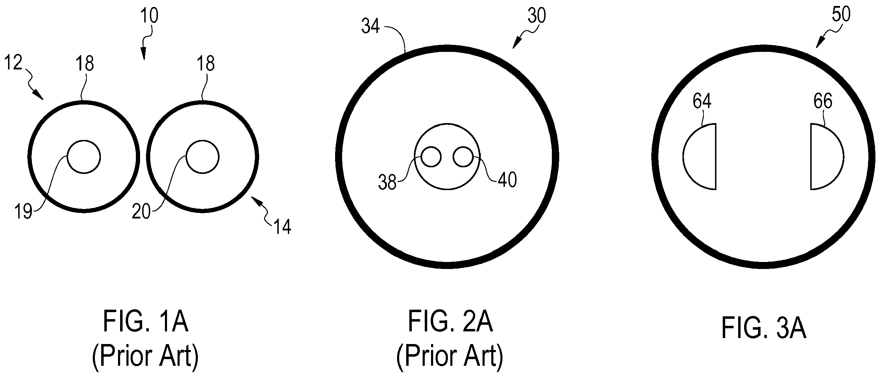

As described above, prior art stereo endoscope optical systems present in two basic configurations including those with two optical systems and those with a single optical system. Referring to FIG. 1, there is depicted a stereo endoscope optical system 10 including two separate optical systems or trains 12, 14. Each of optical systems 12, 14 includes a negative front lens 18 cemented to a piano surface of a plano-convex lens 16 of an objective. As depicted in FIGS. 1 and 1A, use of two separate optical systems 12, 14 provides stereo endoscope optical system 10 with two circular entrance pupils including a left entrance pupil 19 and a right entrance pupil 20 having an extended distance there between. A benefit of stereo endoscope optical system 10 is that it provides adequate right and left perceptive views of the object or working field. A shortcoming of stereo endoscope optical system 10 is that the diameter of the distal end of an endoscope must be great enough to accommodate both optical systems 12, 14.

Referring to FIG. 2, there is depicted a stereo endoscope optical system 30 including a single optical system or train 32. Optical system 32 includes a negative front lens 34 cemented to a piano surface of a plano-convex lens 36 of an objective. As depicted in FIGS. 2 and 2A, a divider 37 functions to provide stereo endoscope optical system 30 with two, relatively small circular entrance pupils including a left entrance pupil 38 and a right entrance pupil 40 having an small distance there between. A benefit of stereo endoscope optical system 30 is that the diameter of the distal end of an endoscope required to accommodate single optical system 32 is smaller than what is required for stereo endoscope optical system 10. A shortcoming of stereo endoscope optical system 30 is that the right and left perceptive views provided of the object or working field of the system are inadequate for some medical and surgical applications because of the proximity of the right and left pupils 38, 40 to one another and the diameter of the pupils.

The present invention relates to a stereo endoscope optical system that overcomes the shortcomings of prior art stereo endoscope optical systems by utilizing a single optical system or train in combination with a pupil expander assembly. The pupil expander assembly divides the single entrance pupil of the single optical train into a left half and a right half and separates the left half from the right by an extended distance. The resulting stereo endoscope optical system can be used in a distal end of a surgical endoscope having a diameter that is as small as the diameter of endoscopes including a conventional, single optical system, while providing perspective right and left views of an object field like what is afforded by endoscope that include two, separate optical systems.

Referring to FIG. 3 there is depicted a stereo endoscope optical system 50 including a single optical train 52 and a pupil expander assembly 54 in accordance with the present invention. Single optical train 52 is defined by relay lens assembly 56 that is optically aligned with an objective including a plano-convex lens 58. Single optical train 52 includes an entrance pupil 59 that is located at a piano surface 60 of the plano-convex objective lens. Supported directly on piano surface 60 is a prism block 62 that functions to separate entrance pupil 59 into a left half 64 and a right half 66 and deflect the light rays sideward.

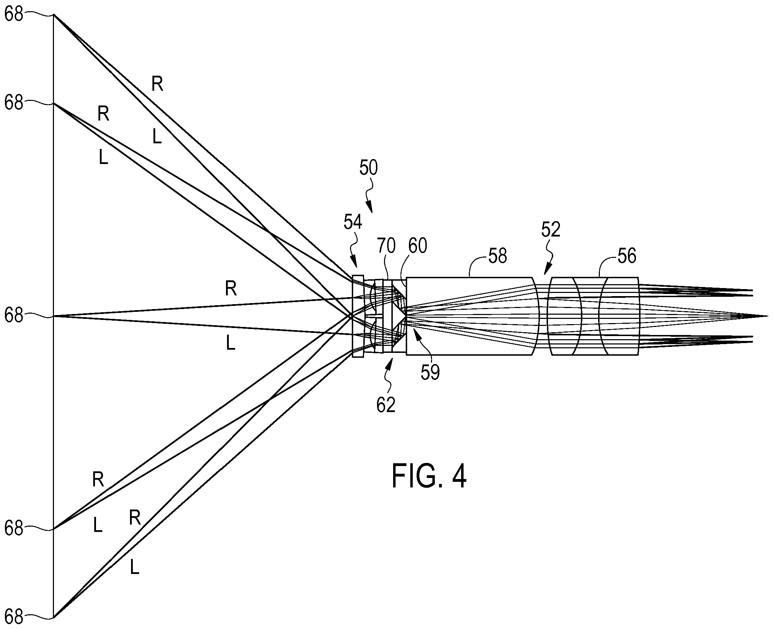

As depicted in FIG. 4, combining single optical train 52 with pupil expander assembly 54 results in a significantly expanded distance between left and right halves 64, 66 of entrance pupil 59, as observed from the object field. Consequently, each object point 68 in the object field is viewed from two perspective points with significant lateral distance. The directions from the left and right perspective points are designated in FIG. 4 with the capital letters L and R.

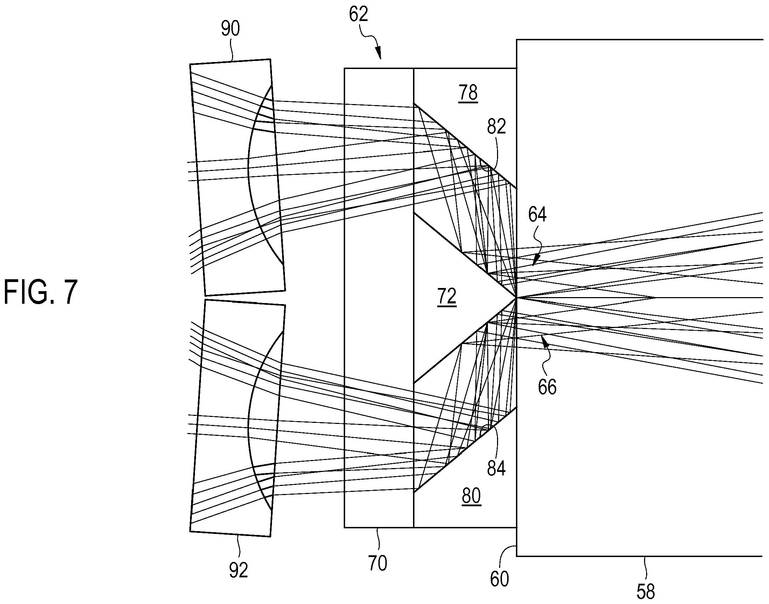

Referring to FIGS 3 and 4, the distance between left and right halves 64, 66 of entrance pupil 59 is expanded by pupil expander 54 and primarily by prism block 62. Prism block 62 includes a glass window 70 cemented to a triangle prism 72. On a left side and a right side of triangle prism 72 are two rhomboidal prisms 74, 76 cemented. Left and right sides of rhomboidal prisms 74, 76 are supported in place by support prisms 78, 80 cemented (prisms 78, 80 not shown in Fig.4 but in Fig. 7).

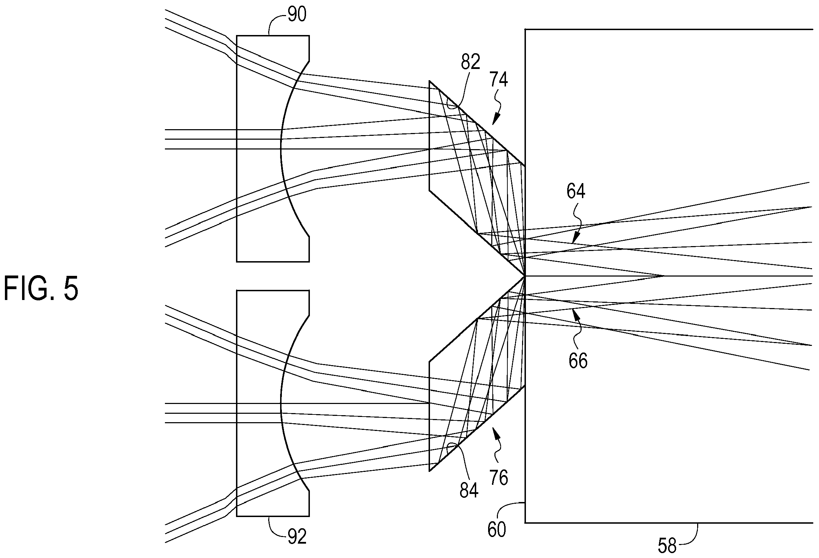

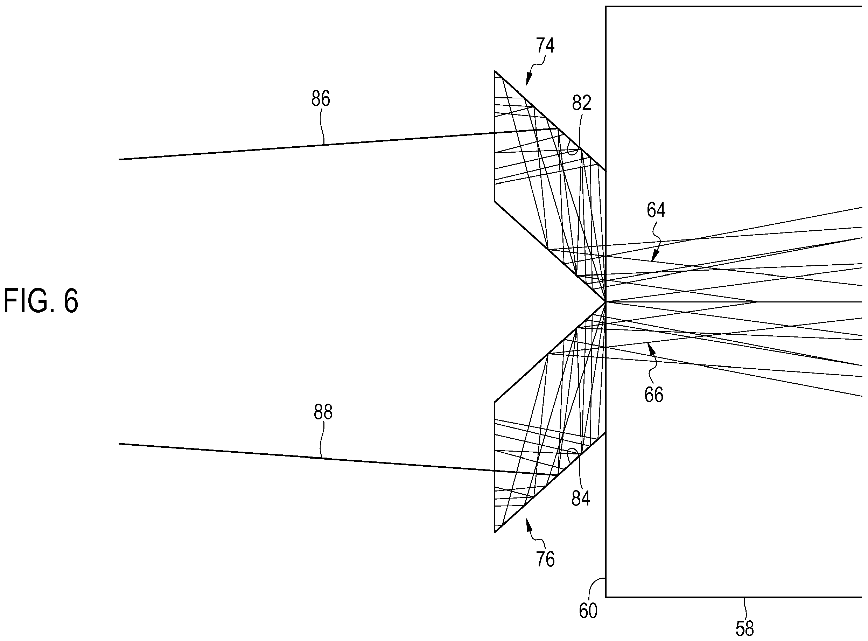

Rhomboidal prisms 74, 76 of prism block 62 divide and separate entrance pupil 59 of the stereo endoscope. As depicted in FIGS. 5 and 6, rhomboidal prisms 74, 76 include respective reflecting sides 82, 84 having slightly different angles which results in the convergence of the two optical axes 86, 88 on the object side.

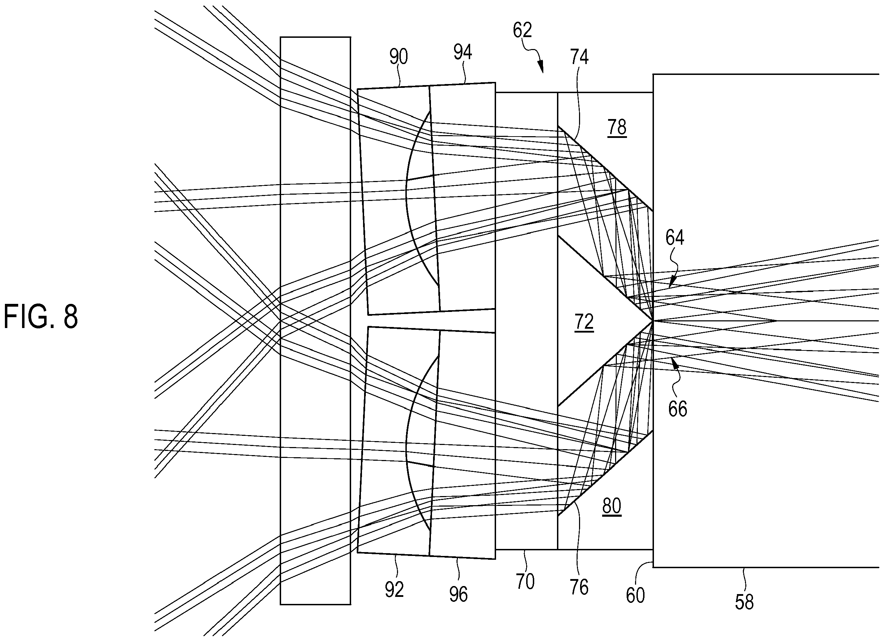

Referring to FIGS. 5 and 7, in front of prism block 62 are located a pair of negative front lenses 90, 92 that are optically aligned with rhomboidal prisms lenses 74, 76, respectively. As depicted in FIG. 8, negative front lenses 90, 92 can be cemented on respective wedged glass plates 94, 96 and aligned in front of and cemented to prism block 62.

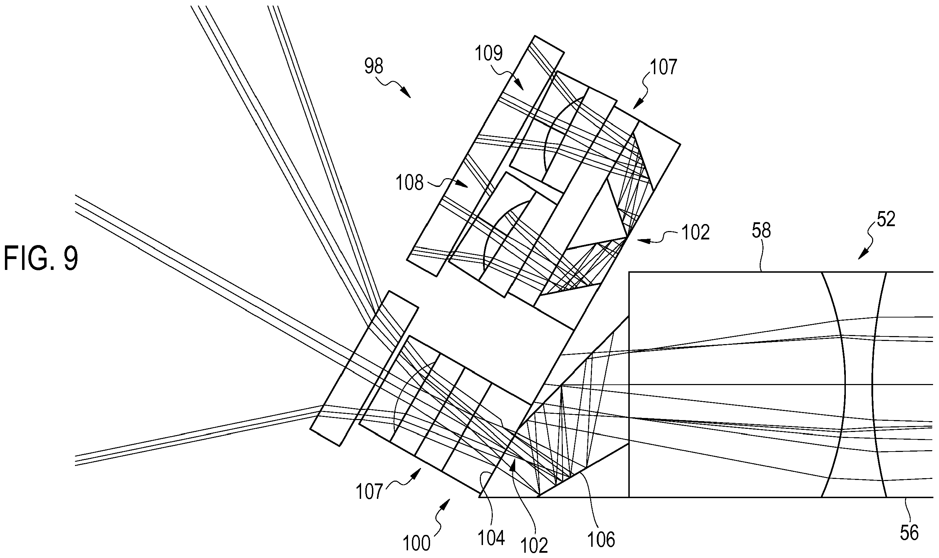

Referring to FIG. 9, there is depicted a stereo endoscope optical system 98 including single optical train 52 and a pupil expander assembly 100 in accordance with the present invention. Single optical train 52 is defined by relay lens assembly 56 that is optically aligned with an objective including plano-convex lens 58. In this embodiment, entrance pupil 102 is located at an exit surface 104 of a deflection prism 106. On exit surface 104 is a prism block 107 cemented which separates entrance pupil 102 into a left portion 108 and a right portion 109 and expands the distance between the two pupil halves.

As will be apparent to one skilled in the art, various modifications can be made within the scope of the aforesaid description. Such modifications being within the ability of one skilled in the art form a part of the present invention and are embraced by the claims below.

## Claims

1. An optical system for a stereo endoscope including a monoscopic endoscope having an entrance pupil that is divided into a left half and a right half thereby creating two stereoscopic images that are observed from a left perspective and a right perspective, the optical system comprising:
an objective lens,
the entrance pupil (102) having an optical axis,
a prism block (62) located adjacent to an entrance pupil side of the objective lens, the prism block (62) including a first rhomboidal prism (74) and a second rhomboidal prism (76),
wherein the first rhomboidal prism has a first corner and the second rhomboidal prism has a second corner, the first corner and the second corner being in contact at a point located on the optical axis of the entrance pupil,
wherein the first rhomboidal prism of the prism block deflects all rays extending through a left half of the entrance pupil to the left and transfers a first stereoscopic image from the left half of the entrance pupil to the left and the second rhomboidal prism of the prism block deflects all rays extending through a right half of the entrance pupil to the right and transfers a second stereoscopic image from the right half of the entrance pupil to the right, **characterised in that**:
the entrance pupil (102) is located at an exit surface of a deflection prism (106), and the prism block is cemented on said exit surface of the deflection prism.

2. The optical system according to claim 1 wherein the first corner is partially defined by a first side that lies directly against the objective lens and the second corner is partially defined by a second side that lies directly against the objective lens (58).

3. The optical system according to claim 1 wherein the objective lens (58) is a plano-convex lens, the entrance pupil being located at a piano surface of the plano-convex lens.

4. The optical system according to claim 1 wherein the point is located immediately adjacent to the entrance pupil (102).

5. The optical system according to claim 1 wherein the prism block (62) includes a triangle shaped prism (72) coupled to and between the first rhomboidal prism (74) and the second rhomboidal prism (76) and optionally wherein the prism block (72) includes a first supporting prism (78) coupled to the first rhomboidal prism and a second supporting prism (80) coupled to the second rhomboidal prism.

6. The optical system according to claim 5 wherein the prism block includes a glass window fixed to the pair of rhomboidal prisms.

7. The optical system according to claim 1 further comprising a first negative lens (90) set being optically aligned with the first rhomboidal prism (74) and a second negative lens set (92) being optically aligned with the second rhomboidal prism (76), and optionally the system comprising a first wedged glass plate (94) optically aligned with and coupled to and between the first negative lens set (90) and the first rhomboidal prism (74) and a second wedged glass plate (96) optically aligned with and coupled to and between the second negative lens set (92) and the second rhomboidal prism (76).

8. The optical system according to claim 7 wherein the negative lens sets are glued on the wedged glass plates and optically aligned with converging optical axes extending from the pair of rhomboidal prisms.

9. The optical system according to claim 1 wherein the first rhomboidal prism (74) has a first reflecting side with a first deflection angle and a second reflecting side with a second deflection angle and the second rhomboidal prism (76) has a third reflecting side with a third deflection angle and a fourth reflecting side a fourth deflecting angle, and optionally wherein the first rhomboidal prism (74) has an optical axis that converges in a working distance with an optical axis of the second rhomboidal prism (76).

10. The optical system according to any preceding claim further comprising a second prism block including a pair of rhomboidal prisms that are located immediately adjacent to an exit pupil of the endoscope objective and arranged to divide the exit pupil in half, and optionally the endoscope further comprising a stereo endoscope camera operatively coupled to a proximal end of the endoscope objective, and optionally the endoscope further comprising an LCD shutter arranged between the exit pupil and the stereo endoscope camera and configured for selectively blocking a left half of the exit pupil and a right half of the exit pupil.

11. A method of using the optical system of any preceding claim comprising dividing the entrance pupil in half with the first and second rhomboidal prisms thereby creating two stereoscopic images, each of the two stereoscopic images being observed from a right perspective and a left perspective.

12. The method according to claim 11 further comprising arranging the first rhomboidal prism and the second rhomboidal prism to have converging optical axes that converge in a working distance.

13. The method according to claim 11 further comprising optically aligning the first negative lens set with the first rhomboidal prism and optically aligning the second negative lens set with the second rhomboidal prism.

14. The method according to claim 11 further comprising forming a prism block with the first rhomboidal prism and the second rhomboidal prism, fixing the prism block to a piano surface of the objective lens wherein the entrance pupil is positioned immediately adjacent to the prism block.

15. The method according to claim 11 further comprising arranging the first rhomboidal prism and the second rhomboidal prism to be immediately adjacent to the entrance pupil.

16. The method according to claim 11 wherein the first rhomboidal prism and the second rhomboidal prism are located at a surface of the entrance pupil.

17. The method according to claim 11 further comprising using the third rhomboidal prism to deflect substantially all rays extending through a first half of an exit pupil of the monoscopic optical system and using the fourth rhomboidal prism to deflect substantially all rays extending through a second half of the exit pupil to the right, and optionally the method further comprising operatively coupling the third rhomboidal prism and the fourth rhomboidal prism between a stereo endoscope camera and the exit pupil.

## Patentansprüche

1. Optisches System für ein Stereo-Endoskop, das ein monoskopisches Endoskop mit einer Eintrittspupille enthält, die in eine linke und eine rechte Hälfte geteilt ist, wodurch zwei stereoskopische Bilder erzeugt werden, die aus einem linken und einem rechten Blickwinkel betrachtet werden, wobei das optische System umfasst:
eine Objektivlinse,
die Eintrittspupille (102) mit einer optischen Achse,
einen Prismenblock (62), der an die Eintrittspupillenseite der Objektivlinse angrenzt, wobei der Prismenblock (62) ein erstes Rhomboidprisma (74) und ein zweites Rhomboidprisma (76) umfasst,
wobei das erste Rhomboidprisma eine erste Ecke und das zweite Rhomboidprisma eine zweite Ecke aufweist, wobei die erste Ecke und die zweite Ecke an einem Punkt in Kontakt sind, der auf der optischen Achse der Eintrittspupille liegt,
wobei das erste Rhomboidprisma des Prismenblocks alle Strahlen, die durch die linke Hälfte der Eintrittspupille verlaufen, nach links ablenkt und ein erstes stereoskopisches Bild von der linken Hälfte der Eintrittspupille nach links überträgt und das zweite Rhomboidprisma des Prismenblocks alle Strahlen, die durch die rechte Hälfte der Eintrittspupille verlaufen, nach rechts ablenkt und ein zweites stereoskopisches Bild von der rechten Hälfte der Eintrittspupille nach rechts überträgt, **dadurch gekennzeichnet, dass**
die Eintrittspupille (102) sich an einer Austrittsfläche eines Ablenkprismas (106) befindet, und der Prismenblock auf diese Austrittsfläche des Ablenkprismas aufgeklebt ist.

2. Optisches System nach Anspruch 1, bei dem die erste Ecke teilweise durch eine erste Seite definiert ist, die direkt an der Objektivlinse anliegt, und die zweite Ecke teilweise durch eine zweite Seite definiert ist, die direkt an der Objektivlinse (58) anliegt.

3. Optisches System nach Anspruch 1, bei dem die Objektivlinse (58) eine plankonvexe Linse ist, wobei die Eintrittspupille an der ebenen Oberfläche der plankonvexen Linse angeordnet ist.

4. Optisches System nach Anspruch 1, bei dem sich der Punkt unmittelbar neben der Eintrittspupille (102) befindet.

5. Optisches System nach Anspruch 1, wobei der Prismenblock (62) ein dreieckförmiges Prisma (72) aufweist, das mit und zwischen dem ersten Rhomboidprisma (74) und dem zweiten Rhomboidprisma (76) gekoppelt ist, und wobei der Prismenblock (72) wahlweise ein erstes Stützprisma (78), das mit dem ersten Rhomboidprisma verbunden ist, und ein zweites Stützprisma (80), das mit dem zweiten Rhomboidprisma verbunden ist, aufweist.

6. Optisches System nach Anspruch 5, wobei der Prismenblock ein Glasfenster aufweist, das an den beiden Rhomboidprismen befestigt ist.

7. Optisches System nach Anspruch 1, ferner einen ersten Negativlinsensatz (90), der optisch mit dem ersten Rhomboidprisma (74) ausgerichtet ist, und einen zweiten Negativlinsensatz (92), der optisch mit dem zweiten Rhomboidprisma (76) ausgerichtet ist, umfassend, und wobei das System wahlweise eine erste keilförmige Glasplatte (94), die optisch mit dem ersten Negativlinsensatz (90) und dem ersten Rhomboidprisma (74) ausgerichtet und mit und zwischen diesen verbunden ist, und eine zweite keilförmige Glasplatte (96) umfasst, die optisch mit dem zweiten Negativlinsensatz (92) und dem zweiten Rhomboidprisma (76) ausgerichtet und mit und zwischen diesen verbunden ist, umfasst.

8. Optisches System nach Anspruch 7, bei dem die Negativlinsensätze auf die keilförmigen Glasplatten geklebt und optisch mit konvergierenden optischen Achsen, die von den beiden Rhomboidprismen ausgehen, ausgerichtet sind.

9. Optisches System nach Anspruch 1, wobei das erste Rhomboidprisma (74) eine erste reflektierende Seite mit einem ersten Ablenkwinkel und eine zweite reflektierende Seite mit einem zweiten Ablenkwinkel aufweist und das zweite Rhomboidprisma (76) eine dritte reflektierende Seite mit einem dritten Ablenkwinkel und eine vierte reflektierende Seite mit einem vierten Ablenkwinkel aufweist, und wobei wahlweise das erste Rhomboidprisma (74) eine optische Achse aufweist, die im Arbeitsabstand mit der optischen Achse des zweiten Rhomboidprismas (76) konvergiert.

10. Optisches System nach einem beliebigen vorhergehenden Anspruch, ferner einen zweiten Prismenblock mit zwei Rhomboidprismen umfassend, die sich unmittelbar neben der Austrittspupille des Endoskopobjektivs befinden und so angeordnet sind, dass sie die Austrittspupille in zwei Hälften teilen, und wobei das Endoskop ferner wahlweise eine Stereo-Endoskopkamera, die operativ mit dem proximalen Ende des Endoskopobjektivs gekoppelt ist, umfasst, und wobei das Endoskop ferner wahlweise einen LCD-Verschluss, der zwischen der Austrittspupille und der Stereo-Endoskopkamera angeordnet und so konfiguriert ist, dass er selektiv die linke Hälfte der Austrittspupille und die rechte Hälfte der Austrittspupille blockiert, umfasst.

11. Verfahren zur Verwendung des optischen Systems nach einem beliebigen vorhergehenden Anspruch, bei dem die Eintrittspupille durch das erste und das zweite Rhomboidprisma in zwei Hälften geteilt wird, wodurch zwei stereoskopische Bilder erzeugt werden, wobei jedes der beiden stereoskopischen Bilder aus einem rechten und einem linken Blickwinkel betrachtet wird.

12. Verfahren nach Anspruch 11, ferner das Anordnen des ersten Rhomboidprismas und des zweiten Rhomboidprismas auf solche Weise umfassend, dass sie konvergierende optische Achsen haben, die im Arbeitsabstand konvergieren.

13. Verfahren nach Anspruch 11, ferner das optische Ausrichten des ersten Negativlinsensatzes mit dem ersten Rhomboidprisma und das optische Ausrichten des zweiten Negativlinsensatzes mit dem zweiten Rhomboidprisma umfassend.

14. Verfahren nach Anspruch 11, ferner das Bilden eines Prismenblocks mit dem ersten Rhomboidprisma und dem zweiten Rhomboidprisma und das Befestigen des Prismenblocks an einer ebenen Oberfläche der Objektivlinse umfassend, wobei die Eintrittspupille unmittelbar angrenzend an den Prismenblock positioniert ist.

15. Verfahren nach Anspruch 11, ferner das Anordnen des ersten Rhomboidprismas und des zweiten Rhomboidprismas in unmittelbarer Nähe der Eintrittspupille umfassend.

16. Verfahren nach Anspruch 11, wobei das erste Rhomboidprisma und das zweite Rhomboidprisma an der Oberfläche der Eintrittspupille angeordnet sind.

17. Verfahren nach Anspruch 11, ferner das Anwenden des dritten rhombischen Prismas, um im Wesentlichen alle Strahlen, die sich durch die erste Hälfte der Austrittspupille des monoskopischen optischen Systems erstrecken, abzulenken, und das Anwenden des vierten rhombischen Prismas, um im Wesentlichen alle Strahlen, die sich durch die zweite Hälfte der Austrittspupille erstrecken, nach rechts abzulenken, umfassend, wobei das Verfahren ferner wahlweise das operative Koppeln des dritten Rhomboidprismas und des vierten Rhomboidprismas zwischen einer Stereo-Endoskopkamera und der Austrittspupille umfasst.

## Revendications

1. Système optique pour un endoscope stéréoscopique comprenant un endoscope monoscopique ayant une pupille d'entrée divisée en une moitié gauche et une moitié droite créant ainsi deux images stéréoscopiques qui s'observent à partir d'une perspective gauche et d'une perspective droite,
le système optique comprenant
- une lentille d'objectif
- une pupille d'entrée (102) ayant un axe optique
- un bloc prismatique (62) adjacent à la lentille d'objectif, côté pupille d'entrée, le bloc prismatique (62) comprenant un premier prisme rhomboïdal (74) et un second prisme rhomboïdal (76),
dans lequel
le premier prisme rhomboïdal a un premier coin et le second prisme rhomboïdal a un second coin, le premier coin et le second coin étant en contact en un point situé sur l'axe optique de la pupille d'entrée,
- le premier prisme rhomboïdal du bloc prismatique dévie tous les rayons traversant la moitié gauche de la pupille d'entrée vers la gauche et transfère une première image stéréoscopique de la moitié gauche de la pupille d'entrée vers la gauche et le second prisme rhomboïdal du bloc prismatique dévie tous les rayons traversant la moitié droite de la pupille d'entrée vers la droite et transfère une seconde image stéréoscopique de la moitié droite de la pupille d'entrée vers la droite,
système **caractérisé en ce que**
la pupille d'entrée (102) est située sur la surface de sortie d'un prisme de déflection (106) et le bloc prismatique est collé sur cette surface de sortie du prisme de déflection.

2. Système optique selon la revendication 1,
dans lequel
le premier coin est partiellement défini par un premier côté qui se situe directement contre la lentille d'objectif et le second coin est partiellement défini par un second côté qui se situe directement contre la lentille d'objectif (58).

3. Système optique selon la revendication 1,
dans lequel
la lentille d'objectif (58) est une lentille plan-convexe, la pupille d'entrée étant située sur la surface plane de la lentille plan-convexe.

4. Système optique selon la revendication 1,
dans lequel
le point est directement adjacent à la pupille d'entrée (102).

5. Système optique selon la revendication 1,
dans lequel
le bloc prismatique (62) comprend un prisme de forme triangulaire (72) couplé au premier prisme rhomboïdal (74) entre celui-ci et le second prisme rhomboïdal (76) et en option
le bloc prismatique (72) comprend un premier prisme de support (78) couplé au premier prisme rhomboïdal et un second prisme de support (80) couplé au second prisme rhomboïdal.

6. Système optique selon la revendication 5,
dans lequel
le bloc prismatique comprend une fenêtre de verre fixée à la paire de prisme rhomboïdaux.

7. Système optique selon la revendication 1,
comprenant en outre une première lentille divergente (90) fixée pour être alignée optiquement sur le premier prisme rhomboïdal (74) et une seconde lentille divergente (92) alignée optiquement sur le second prisme rhomboïdal (76) et en option
le système comprend une première plaque de verre formant un coin (94) alignée optiquement et couplée entre la première lentille divergente (90) et le premier prisme rhomboïdal (74) ainsi qu'une seconde plaque de verre formant un coin (96) alignée optiquement et couplée entre la seconde lentille divergente (92) et le second prisme rhomboïdal (76).

8. Système optique selon la revendication 7,
selon lequel
les lentilles divergentes sont collées sur les plaques de verre en forme de coin et sont alignées optiquement sur les axes optiques de convergence partant de la paire de prismes rhomboïdaux.

9. Système optique selon la revendication 1,
dans lequel
le premier prisme rhomboïdal (74) a un premier côté réfléchissant avec un premier angle de déflection et un second côté réfléchissant avec un second angle de déflection et
le second prisme rhomboïdal (76) a un troisième côté réfléchissant avec un troisième angle de déflection et un quatrième côté réfléchissant avec un quatrième angle de déflection et en option
le premier prisme rhomboïdal (74) a un axe optique convergeant à une distance utile avec l'axe optique du second prisme rhomboïdal (76).

10. système optique selon l'une quelconque des revendications précédentes comprenant en outre
un second bloc prismatique comprenant une paire de prismes rhomboïdaux directement adjacents sur une pupille de sortie de l'objectif de l'endoscope et organisés pour diviser la pupille de sortie en moitiés et en option
l'endoscope comporte en outre une caméra d'endoscope stéréoscopique coopérant avec une extrémité proximale de l'objectif d'endoscope et en option
l'endoscope comprend en outre un obturateur LCD entre la pupille de sortie et la caméra d'endoscope stéréoscopique et configuré pour bloquer sélectivement une moitié gauche de la pupille de sortie et une moitié droite de la pupille de sortie.

11. Procédé d'utilisation du système optique selon l'une quelconque des revendications précédentes consistant à,
diviser la pupille d'entrée en moitiés avec le premier et le second prisme rhomboïdal en créant ainsi deux images stéréoscopiques, chacune des deux images stéréoscopiques s'observant à partir d'une perspective à droite et d'une perspective à gauche.

12. Procédé selon la revendication 11, consistant en outre à :
- arranger le premier prisme rhomboïdal et le second prisme rhomboïdal pour que les axes optiques convergent à une distance utile.

13. Procédé selon la revendication 11 consistant en outre à
- aligner optiquement la première lentille divergente sur le premier prisme rhomboïdal et aligner optiquement la seconde lentille divergente sur le second prisme rhomboïdal.

14. Procédé selon la revendication 11 consistant en outre à
- former un bloc prismatique avec le premier prisme rhomboïdal et le second prisme rhomboïdal et
- fixer le bloc prismatique sur la surface plane de la lentille d'objectif,
la pupille d'entrée étant positionnée de façon directement adjacente au bloc prismatique.

15. Procédé selon la revendication 11 consistant en outre à
- arranger le premier prisme rhomboïdal et le second prisme rhomboïdal pour qu'ils soient directement adjacents à la pupille d'entrée.

16. Procédé selon la revendication 11,
selon lequel
- le premier prisme rhomboïdal et le second prisme rhomboïdal sont situés sur la surface de la pupille d'entrée.

17. Procédé selon la revendication 11 consistant en outre à
- utiliser le troisième prisme rhomboïdal pour dévier pratiquement tous les rayons traversant une première moitié d'une pupille de sortie du système optique monoscopique et
- utiliser le quatrième prisme rhomboïdal pour dévier pratiquement tous les rayons traversant une seconde moitié de la pupille de sortie vers la droite et
en option le procédé consiste en outre à :
- coupler de façon à coopérer, le troisième prisme rhomboïdal et le quatrième prisme rhomboïdal entre une caméra d'endoscope stéréoscopique et la pupille de sortie.
